# EUROPEAN PATENT APPLICATION

(11) **EP 1 634 590 A1**
(43) Date of publication of application: **15.03.2006**
(21) Application number: 04748924.0
(22) Date of filing: 03.06.2004
(51) Int. Cl.: A61K 31/165, A61P 31/14

(54) **CORONAVIRUS INACTIVATING AGENT**

(30) Priority: 04.06.2003 RU 2003116391
(71) Applicant: Megainpharm GmbH, 1020 Wien (AT)
(72) Inventor: KRIVOSHEIN, Yuru Semionovich, Moscow, 103045 (RU); RUDKO, Adolina Petrovna, Moscow, 113054 (RU)
(74) Representative: Jeck, Anton
(86) International application number: PCT/RU2004/000215
(87) International publication number: WO 2004/108125

(57) **Zusammenfassung**

Die Erfindung betrifft einen neuen Koronavirus-Inaktivierungswirkstoff in Form von Miramistin (d.i. Myristamid-Propyl-Dimethyl-Benzyl-Ammoniumchlorid), das früher als Antizeptionsmittel bekannt war, dabei antimykotische und antimikrobielle Wirkungen verhinderte und wirksam gegen HIV-, Influenza- und Herpesvirusinfektionen ist. Die Erfindung erweitert den Bereich der Wirkstoffe für die genannten Verwendungen und ermöglicht die Behandlung und die Verhinderung von Krankheiten des Atmungstrakts (einschließlich SARS) und von Gastroenteritis, die durch die genannten Koronaviren hervorgerufen werden.

## Description

Die Erfindung gehört zu Medizinbereich und Pharmaindustrie und kann in der Entwicklung, Herstellung und Anwendung der Heilmittel und prophylaktischer Präparate eingesetzt werden.

Bekannt ist der Einsatz von Fettlösungsmitteln, Detergentien und Antiseptiken als Inaktivierungsmittel der Koronaviren (L.J.Zakstelskaja, A.W. Scheboldow. Koronaviren des Menschen und der Tiere. Moskau, Verlag Medizin, 1977, S. 221. Allgemeine und spezielle Virusforschung. Unter Redaktion von W.M. Schdanow, S.J. Gaidamowitsch. 1982, Moskau, Verlag Medizin, Band 2, S. 316-339. A.J.Korotajew, S.A. Babitschew. Medizinische Mikrobiologie, Virus- und Immunitätsforschung. St-Petersburg, Verlag Fachliteratur. 1998, S. 273).

Erfindungsgemäß wird vorgeschlagen, zur Inaktivierung der Koronaviren Myristamidopropyldimethylbenzylammoniumchlorid einzusetzen, das unter dem Namen Myramistin (Urheberzeugnis 1832496) bis jetzt als Präventivmittel gegen konventionelle venerische Krankheiten wie Syphilis, Trichomoniasis und Tripper bekannt ist und als Antiseptikum (Patent RU 2164135) zur Behandlung der eitrigen Korneaentzündung eingesetzt wird.

Die anzumeldende technische Lösung ermöglicht, den Vorrat an Mitteln zur Prophylaxe und Behandlung der Krankheiten zu erweitern, die von den Koronaviren hervorgerufen werden.

Inaktivierende Wirkung des anzumeldenden Mittels wird durch folgende Beispiele veranschaulicht (zur Vereinfachung der Darlegung im weiteren Myramistin genannt).

### Beispiel 1:

Ermittlung der minimalen Hemmungskonzentration (MHK) von Myramistin wurde in bezug auf Testkultur der Koronaviren (HCoV OC43) in der Nierenzellkultur des Humanembryos in vitro nach folgendem Plan durchgeführt:

| | |
|---|---|
| 1.Schritt | Testvirus + Myramistin (in vorgegebener Konzentration) |
| 2.Schritt | Inkubation (2 Stunden) |
| 3.Schritt | Zugabe eines Neutralisierungsmittels zum Gemisch (z.B. 25 % Kuhembryo-Serum) und Inkubation 10-20 Min. |
| 4.Schritt | Ansteckung der sensiblen Zellkultur (z.B. Nieren des Humanembryos) |
| 5.Schritt | Inkubation (5-8 Tage) |
| 6.Schritt | Erfassung der Ergebnisse nach dem Hämadsorption-Phänomen und zytopathischer Wirkung - Symplasmabildung |

Die Versuchsreihen wurden mit vier unterschiedlichen Konzentrationen des Präparates ausgeführt, danach wurden seine MHK-Werte in Bezug auf die Koronaviren berechnet. Versuchsergebnisse sind in der Tabelle 1 dargestellt.

**Tabelle 1 MHK von Myramistin und anderen Aniseptiken in Bezug auf HCoV OC43 in vitro**

| *Antiseptikum* | *MHK (%)* |
|---|---|
| Myramistin | 0,01 ± 0,005 |
| Decametoxin | 0,1 ± 0,05 |
| Chlorhexidin | 0,05 ± 0,005 |
| Etonium | 0,5 ± 0,2 |
| Dioxidin | R |
| Roccal | 0,1 ± 0,05 |
| Nonoxynol 9 | 0,2 ± 0,09 |
| Tween 20 | 2,0 ± 0,8 |

Anmerkung: R = gegen Antiseptikumkonzentration über 2,5 % resistent.

Wie aus den Daten Tabelle 1 ersichtlich ist, übertrifft Myramistin in der Inaktivierungswirkung auf die Koronaviren die zur Zeit bekannten und anzuwendenden Antiseptiken.

### Beispiel 2:

Ermittlung von Myramistin-MHK wurde in Bezug auf HCoV OC 43 bei Maussaugern in vivo nach folgendem Plan durchgeführt:

| | |
|---|---|
| 1.Schritt | Testvirus + Myramistin (in vorgegebener Konzentration) |
| 2.Schritt | Inkubation (30 Min.) |
| 3.Schritt | Ansteckung der Maussauger durch Gehirn bzw. Bauchraum |
| 4.Schritt | Erfassung der Ergebnisse am 2.-3. Tag nach der Ansteckung (Hyperämie des Hautüberzuges, Tremor, Muskelsteifheit, kein Saugen, Tod der Tiere) |

Insgesamt wurden 3 Versuchsreihen mit 4 Konzentrationen und je 6 Tieren durchgeführt. Vireninfektionsfähigkeit bei Maussaugern wurde in IgLD₅₀ ausgedrückt. Die durchschnittlichen Versuchsergebnisse stellt die Tabelle 2 dar.

**Tabelle 2 Untersuchungsergebnisse von Myramistin-MHK in Bezug auf Infektionsfähigkeit HCoV OC 43 bei Maussaugern in vivo**

| Reihen-Nr. | Infektionsaktivität der Viren (in IgLD₅₀) | | | | |
|---|---|---|---|---|---|
| | vor Behandlung | nach Behandlung mit Dose (in %) | | | |
| | | 0,0001 | 0,001 | 0,005 | 0,01 |
| 1. | 6,4 | 3,6 | 1,0 | 0 | 0 |
| 2. | 6,0 | 3,2 | 1,0 | 0 | 0 |
| 3. | 6,3 | 3,0 | 1,2 | 0 | 0 |

Bewertung der erhaltenen Daten zeigt, daß Myramistin in der Konzentration von 0,0001 % Infektionsaktivität der Koronaviren fast um das Doppelte herabsinkt und in der Konzentration von 0,005 % und mehr völlig neutralisiert.

### Beispiel 3: Myramistin-Einfluß auf Hämagglutinationsaktivität der Koronaviren OC 43 wurde durch Hämagglutinationsreaktion mit Huhn-Erythrozyten festgestellt.

Zu der HCoV OC 43 mit Hämagglutinationstiter min. 1:512 enthaltenen Kulturflüssigkeit Myramistin-Lösung in der Konzentration zwischen 0,000001 und 0,001 % geben, das Gemisch im Laufe von 30 Min. inkubieren, 25 % Kuhembryo-Serum zur Neutralisierung von Myramistin zugeben, 10-20 Min. stehen lassen, dann Hämagglutinationsreaktion mit Huhn-Erythrozyten durchführen. Insgesamt wurden 4 Versuchsreihen mit 4 Konzentrationen und je 3 Versuchen durchgeführt. Durchschnittsergebnisse sind in der Tabelle 3 dargestellt.

**Tabelle 3 Versuchsergebnisse der Myramistin-Wirkung auf Hämagglutinationsaktivität der Koronaviren OC 43**

| Reihen-Nr. | Hämagglutinationsaktivität im Titer | | | | |
|---|---|---|---|---|---|
| | vor Behandlung | nach Behandlung mit Dose (in %) | | | |
| | | 0,000001 | 0,0001 | 0,0001 | 0,001 |
| 1. | 512 | 8 | 4 | 0 | 0 |
| 2. | 2048 | 16 | 8 | 0 | 0 |
| 3. | 1024 | 8 | 4 | 0 | 0 |
| 4. | 2560 | 16 | 8 | 0 | 0 |

Versuchsergebnisse zeigen, daß Myramistin auf Hämagglutinin der Koronaviren OC 43, das ihre Adsorption in den sensiblen Zellen gewährleistet, in den niedrigeren Konzentrationen (0,000001 %) zu wirken beginnt als auf ihre Infektionseigenschaften (0,0001 %).

Aus den Beispielen ist ersichtlich, dass die anzumeldende technische Lösung die Verbindung von Myristamidopropyldimethylbenzylammoniumchlorid (Myramistin) als Inaktivierungsmittel der Koronaviren einsetzen und auf deren Basis neue prophylaktische Präparate und Heilmittel zur Bekämpfung der Krankheiten entwickeln lässt, die von Koronaviren hervorgerufen werden.

## Patentansprüche

1. Verwendung von Myristamidopropyldimethylbenzylammoniumchlorid (Myramistin) als Inaktivierungsmittel der Koronaviren.
